(19) Europäisches Patentamt
European Patent Office
Office européen des brevets

(11) Numéro de publication: **0 225 217 A1**

(12) **DEMANDE DE BREVET EUROPEEN**

(21) Numéro de dépôt: **86402343.7**

(22) Date de dépôt: **20.10.86**

(51) Int. Cl.[4]: **C 07 C 49/825**
C 07 C 45/46, C 07 C 45/54,
C 07 D 311/22

(30) Priorité: **21.10.85 FR 8515564**

(43) Date de publication de la demande:
**10.06.87 Bulletin 87/24**

(84) Etats contractants désignés:
**AT BE CH DE ES FR GB GR IT LI LU NL SE**

(71) Demandeur: **ISOCHEM (société anonyme)**
**10, rue Clément Marot**
**F-75008 Paris (FR)**

(72) Inventeur: **Wirth, Didier**
**3, Place du Palais Bourbon**
**F-75007 Paris (FR)**

**Gibert, Dominique**
**12, rue de l'Eglise**
**F-60340 Villers-s'Saint-Leu (FR)**

(74) Mandataire: **L'Helgoualch, Jean**
**OFFICE PICARD 134 Boulevard de Clichy**
**F-75018 Paris (FR)**

(54) **(Chloro-3 propionyl)-2 fluoro-4 phénol, procédé pour sa préparation, et application à la préparation de la fluoro-6 chromanone-4.**

(57) L'invention le (chloro-3 propionyl)-2 fluoro-4 phénol de formule générale (I) :

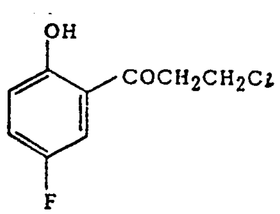

(I)

que l'on prépare par acylation de Friedel et Crafts du fluoro-4 phénol ou d'un de ses éthers par le chlorue de chloro-3 propionyle, ou bien par réaction de transposition de Fries sur le chloro-3 propionate de fluoro-4 phényle.

Application à la préparation de la fluoro-6 chromanone-4 de formule générale (II) :

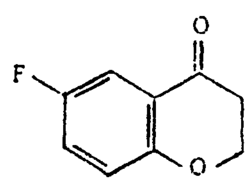

(II)

par cyclisation du (chloro-3 propionyl)-2 fluoro-4 phénol à température ambiante ou par simple chauffage.

EP 0 225 217 A1

## Description

(Chloro-3 propionyl)-2 fluoro-4 phénol procédé pour sa préparation, et application à la préparation de la fluoro-6 chromanone-4

La présente invention se rapporte au (chloro-3 propionyl)-2 fluoro-4 phénol, à un procédé pour sa préparation, à son application à la préparation de la fluoro-6 chromanone-4 ainsi qu'à un procédé de préparation de la fluoro-6 chromanone-4 par l'intermédiaire du (chloro-3 propionyl)-2 fluoro-4 phénol.

La fluoro-6 chromanone-4 est un composé connu, utilisé dans la synthèse industrielle d'inhibiteurs d'aldose réductase tels que le sorbinil (DCI).

La fluoro-6 chromanone-4 est classiquement obtenue par cyanoéthylation du fluoro-4 phénol suivie d'hydrolyse et de cyclisation: l'acrylonitirle utilisé dans la première étape est un produit toxique et de manipulation délicate du fait de sa volatilité et de sa grande tendance à la polymérisation; un autre inconvénient majeur de cette synthèse est son rendement médiocre, de l'ordre de 65% dans les meilleurs cas.

Il est donc particulièrement important de pouvoir disposer d'un procédé utilisant des composés de faible toxicité, et procurant le produit recherché avec un meilleur rendement.

Il a maintenant été découvert que la fluoro-6 chromanone-4 pouvait être obtenue de façon simple et sûre et avec un excellent rendement par cyclisation du (chloro-3 propionyl)-2 fluoro-4 phénol qui peut lui même être préparé à partir de matières premières facilement accessibles.

Les brevets EP 0.031.795 et EP 0.063.799 décrivent des dérivés de la butyrophénone; d'autres dérivés voisins sont également décrits dans le brevet US 3.784.643 et par R. Martin, Bull. Soc. Chim. n° 2 (1985) p.239-242. Toutefois, ces documents concernent des applications différentes de l'invention et n'envisagent pas la préparation de la fluoro-chromanone

La présente invention a donc pour objet le (chloro-3 propionyl)-2 fluoro-4 phénol, ainsi qu'un procédé permettant de la préparer dans de bonne conditions et avec un bon rendement.

L'invention a également pour objet l'application du (chloro-3 propionyl)-2 fluoro-4 phénol à préparation de la fluoro-6 chromanone-4 par cyclisation à température ambiante ou par simple chauffage.

Enfin, l'invention a pour objet un procédé de préparation de la fluoro-6 chromanone-4 à partir de composés facilement accessibles, avec un rendement satisfaisant et en évitant l'utilisation de produits toxiques.

Le (chloro-3 propionyl)-2 fluoro-4 phénol peut être représenté par la formule (I) ci-après :

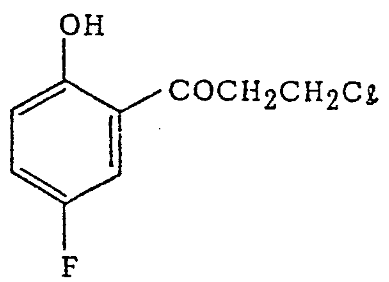

(I)

Le (chloro-3 propionyl)-2 fluoro-4 phénol est un corps nouveau facilement accessible à partir du fluoro-4 phénol ou d'un éther du celui-ci, tel que l'anisole, par réaction d'acylation de Friedel et Crafts à l'aide du chlorure de chloro-3 propionyle selon le schéma suivant :

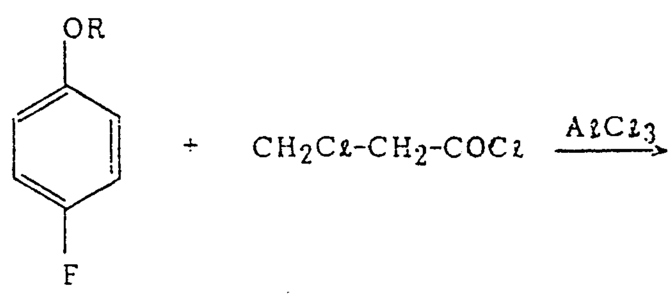


(I)

où R représente un atome d'hydrogène ou un radical alcoylé, de préférence méthyle.

On peut aussi transposer par la réaction de Fries, le chloro-3 propionate de fluoro-4 phényle suivant le schéma suivant :

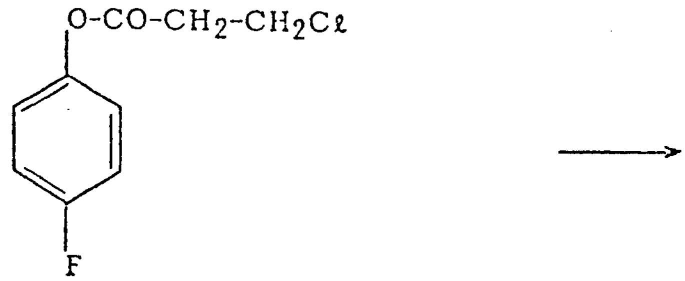

( I )

Le fluoro-4 phénol et ses éthers, de même que le chlorure de chloro-3 propionyle sont des substances disponibles industriellement. Dans les deux cas, le passage au (chloro-3 propionyl)-2 fluoro-4 phénol recherché, s'effectue avec un rendement supérieur à 80%; le produit est obtenu avec une pureté suffisante pour rendre son isolement le plus souvent inutile.

Le (chloro-3 propionyl)-2 fluoro-4 phénol ainsi obtenu peut être appliqué à la préparation de la fluoro-6 chromanone-4 de formule (II) :

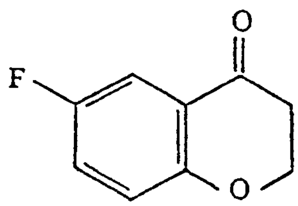

( II )

par cyclisation par simple chauffage, ou à température ambiante.

On peut effectuer cette transformation dans des conditions très variées, de préférence dans un solvant qui peut être choisi parmi les hydrocarbons, aromatiques ou non, tels que le toluène ou l'heptane, des alcools comme le méthanol, l'éthanol, l'isopropanol ou les butanols, des esters comme les acétates d'éthyle, isopropyle ou butyle, les solvants chlorés tels que le chloroform, le dichlorométhane, le dichloroéthane ou le chlorbenzène, les acides, l'eau, isolément ou en mélange.

L'utilisation d'un accepteur de l'acide chlorohydrique libéré peut se révéler bénéfique sans toutefois être nécessaire; on utilise de préférence les hydroxydes ou les sels d'acides faibles des métaux alcalins ou alcalino terreux ou encore les amines.

La température optimale dépend à la fois du solvant et de la base employés : pour des raisons de commodité industrielle on opère souvent à la température de reflux, mais il est également possible, dans certains cas, d'effectuer la réaction à température ambiante, et dans ce cas de préférence en milieu basique, par exemple en présence d'un hydroxyde ou d'un sel de métal alacalin. Le choix de la température en fonction du solvant et, le cas échéant, de l'accepteur d'acide, est à la portée de l'homme du métier.

Le rendement de la cyclisation est sensiblement quantitatif, ce qui permet d'obtenir un produit brut d'excellente qualité ne contenant que des traces d'oligomères; une purification ultérieure peut être effectuée si nécessaire par des moyens connus tels que le recristallisation, la distillation ou autres.

La littérature chimique ne mentionne que très peu de tentatives de cyclisation en chromanones des ortho-(chloro-3 propionyl) phénols : ainsi F. MAYER (Chem. Ber. 57, 1924, p 200) obtient l'hydroxy-7 indanone-1 par action du chlorure d'aluminium sur l'ortho-(chloro-3 propionyl) phénol et non la chromanone espérée. Par ailleurs, l'action de l'acétate de sodium ou du méthylate de sodium sur l'ortho-(chloro-3 propionyl) diméthoxy-4,6 phénol conduit (J.A. DONNELLY, Tetrahedron 35, 1979, p 2883) respectivement aux cétones insaturées ou méthoxylées mais non à la diméthoxy chromanone que l'on aurait pu attendre.

On constate donc que le (chloro-3 propionyl)-2 fluoro-4 phénol selon l'invention est particulièrement utile en permettant de réaliser une synthèse industrielle fort économique et d'une efficacité assez surprenante sur le plan chimique, de la fluoro-6 chromanone-4 dont l'importance industrielle est bien connue.

Les exemples suivants illustrent l'invention sans en limiter la portée.

EXEMPLE 1

(chloro-3 propionyl)-2 fluoro-4 phénol

Dans un réacteur de 2 litres on charge successivement et sans dépasser une température de 30°C 1 litre de dichlor-1,2 éthane, 320g de chlorure d'aluminium, 210ml de chlorure de chloro-3 propionyle et 252g de fluoro-4 anisole pluis on agite le mélange à température modérée pendant 15 heures.

On chauffe ensuite à 40°C environ pendant 4 heures, soit jusqu'à la fin du dégagement de chlorure de méthyle.

On hydrolyse à l'aide de 2 litres d'eau; la phase organique est lavée à l'eau puis concentrée sous vide

moyen. Après reprise à l'heptane et séchage, on obtient 352g de produit beige clair, soit un rendement de 87%.
. Pureté (HPLC) = 99,4%
. F = 63°C

EXEMPLE 2

Fluoro-6 chromanone-4
On chauffe à l'ebullition pendant 30mn un mélange de 2g de composé obtenu selon l'exemple 1 et 10g d'eau; l'analyse montre que l'on obtient un mélange de 80% de produit de départ et 20% de fluoro-6 chromanone-4.

EXEMPLE 3

Fluoro-6 chromanone-4
On agite pendant 1 heure à température ambiante un mélange de 5g de (chloro-3 propionyl)-2 fluoro-4 phénol, 2g de soude et $50cm^3$ de méthanol. Après addition de $100cm^3$ d'eau pour provoquer la précipitation du produit, on obtient 3,8g de fluoro-6 chromanone-4 soit un rendement de 93%. . F = 114°C

EXEMPLE 4

Fluoro-6 chromanone-4
On porte au reflux pendant 30mn un mélange de 160g de (chloro-3 propionyl)-2 fluoro-4 phénol, 272g de carbonate de potasium et $640cm^3$ d'eau. Après refroidissement à 30° C, on filtre, lave à l'eau et sèche.
On obtient ainsi 127g de fluoro-6 chromanone-4 soit un rendement de 97%.
. F = 114°C
. Pureté (HPLC) = 98,5%

**Revendications**

1. Le (chloro-3 propionyl)-2 fluoro-4 phénol de formule générale (I) :

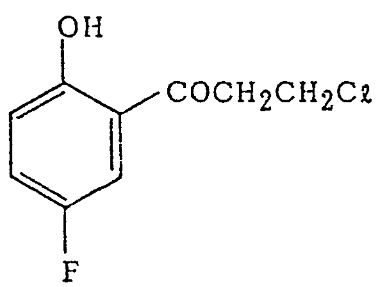

( I )

2. Procédé de préparation du (chloro-3 propionyl)-2 fluoro-4 phénol selon la revendication 1, caractérisé en ce que :
- on effectue une acylation de Friedel et Crafts du fluoro-4 phénol ou d'un de ses éthers par le chlorure de chloro-3 propionyle, ou bien
- on effectue une réaction de transposition de Fries sur le chloro-3 propionate de fluoro-4 phényle.
3. Application du (chloro-3 propionyl)-2 fluoro-4 phénol selon la revendication 1 à la préparation de la fluoro-6 chromanone-4 de formule générale (II) :

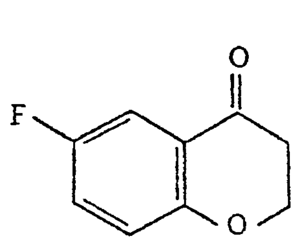

( II )

caractérisé en ce qu'on effectue un cyclisation du (chloro-3 propionyl)-2 fluoro-4 phénol à température ambiante ou par simple chauffage.

4. Procédé de préparation de la fluoro-6 chromanone-4 de formule générale (II) :

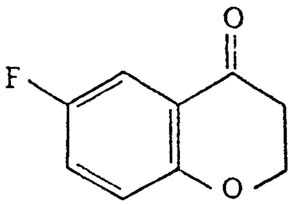

( II )

caractérisé en ce que :
- on effectue une acylation de Friedel et Crafts du fluoro-4 phénol ou d'un de ses éthers par le chlorure de chloro-3 propionyle. ou bien
- on effectue une réaction de transposition de Fries sur le chloro-3 propionate de fluoro-4 phényle pour former le (chloro-3 propionyl)-2 fluoro-4 phénol de formule générale (I) :

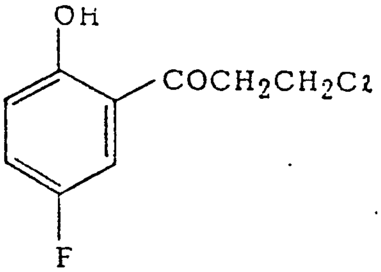

( I )

puis on effectue une cyclisation par simple chauffage ou à température ambiante.

5. Procédé selon la revendication 4, caractérisé en ce que la réaction de cyclisation du (chloro-3 propionyl)-2 fluoro-4 phénol s'effectue dans un solvant en chauffant à reflux.

6. Procédé selon l'une quelconque des revendications 4 et 5, caractérisé en ce que la réaction de cyclisation s'effectue en présence d'un accepteur d'acide.

7. Procédé selon la revendication 6, caractérisé en ce que l'accepteur d'acide est choisi parmi un hydroxyde de métal alcalin ou alcalino-terreux, un sel d'acide faible de métal alcalin ou alcalino-terreux et une amine.

8. Procédé selon la revendication 4, caractérisé en ce que la réaction de cyclisation du (chloro-3 propionyl)-2 fluoro-4 phénol s'effectue à température ambiante en milieu basique.

9. Procédé selon l'une quelconque des revendications 5 et 8, caractérisé en ce que le solvant est choisi parmi un hydrocarbure aromatique, un alcohol, un ester d'alkyle, un solvant chloré, un acide et l'eau, isolément ou en mélange.

Office européen des brevets

# RAPPORT DE RECHERCHE EUROPEENNE

Numero de la demande

EP 86 40 2343

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl 4) |
|---|---|---|---|
| D,Y | EP-A-0 031 795 (CIBA-GEIGY) * Pages 10,11,27,29,30 * | 1,2 | C 07 C 49/825<br>C 07 C 45/46<br>C 07 C 45/54<br>C 07 D 311/22 |
| D,Y | EP-A-0 063 799 (HOECHST-ROUSSEL PHARMACEUTICALS INC) * Page 32, exemple 12 * | 1,2 | |
| D,Y | US-A-3 784 643 (J.T. SUH et al.) * Exemple 15 * | 1,2 | |
| D,Y | BULLETIN DE LA SOCIETE CHIMIQUE DE FRANCE, no. 2, 1985, pages 239-242; R. MARTIN: "Isolement et identification des impuretés formées au cours de la synthèse industrielle de la fluoro-5 hydroxy-2 butyrophénone par transposition de Fries" * Page 239 * | 1,2 | |

DOMAINES TECHNIQUES RECHERCHES (Int. Cl.4)

C 07 C 49/00
C 07 C 45/00

Le présent rapport de recherche a été établi pour toutes les revendications

| Lieu de la recherche | Date d'achèvement de la recherche | Examinateur |
|---|---|---|
| LA HAYE | 27-01-1987 | BONNEVALLE E.I.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82